# EUROPEAN PATENT APPLICATION

(11) **EP 2 915 535 A1**
(43) Date of publication of application: **09.09.2015**
(21) Application number: 13851726.3
(22) Date of filing: 05.11.2013
(51) Int. Cl.: A61K 31/7032, A61K 9/127, A61K 39/395, A61K 45/00, A61P 37/06, A61P 43/00

(54) **IMMUNE-TOLERANCE INDUCER**

(30) Priority: 05.11.2012 JP 2012243967
(71) Applicant: Regimmune Corporation, Tokyo, 1030001 (JP); Tokyo Women's Medical University, Tokyo 162-8666 (JP)
(72) Inventor: HIRAI, Toshihito, Tokyo 162-8666 (JP); OMOTO, Kazuya, Tokyo 162-8666 (JP); TANABE, Kazunari, Tokyo 162-8666 (JP); KAWAGUCHI, Emi, Tokyo 103-0001 (JP); ISHII, Yasuyuki, Tokyo 103-0001 (JP); MORITA, Haruhiko, Tokyo 103-0001 (JP)
(74) Representative: Müller-Boré & Partner Patentanwälte PartG mbB
(86) International application number: PCT/JP2013/079865
(87) International publication number: WO 2014/069655

(57) **Abstract**

The purpose of the present invention is to create an immune tolerance-inducing agent used in therapy in which donor hematopoietic cells are transplanted into a recipient in order to induce immune tolerance in the recipient with respect to donor cells, tissue, or organs. By using an alpha-galactosylceramide-containing liposome in combination with a costimulatory-pathway-blocking substance, hematopoietic chimerism can be induced in the recipient by transplantation of donor hematopoietic cells, making it possible to induce immune tolerance in the recipient with respect to donor cells, tissue, or organs.

## Description

### TECHNICAL FIELD

The present invention relates to an immune tolerance-inducing agent used in a therapy in which donor hematopoietic cells are transplanted into a recipient in order to induce immune tolerance in the recipient with respect to donor cells, tissue, or organ.

### BACKGROUND ART

Organ transplantation is recognized as the final and sole radical treatment for many organ failures. Due to developments and advances of immunosuppressants, it has become possible to control many acute rejections of the transplanted organs. On the other hand, due to toxicity of long-term administration of an immunosuppressant or a chronic rejection that is difficult to be controlled by current immunosuppressants, it cannot still be said that the long-term survival rate of the transplanted organ is good. Induction of transplantation tolerance aims to allow the immune system of an organ transplant recipient to recognize the transplanted organ as autologous, and it is considered that, once this immune tolerance is induced, it is possible to markedly reduce administration of immunosuppressants, and even chronic rejection can be controlled. For induction of immune tolerance, many research results have been reported, and some clinical applications have also been currently reported. However, the mechanism thereof has yet been unclear, and a safe and reliable method has not been established.

Ildostad et al. have reported that, in experiments with rats, bone marrow transplantation enriched with graft survival promoting cells (facilitating cells) in the donor bone marrow is performed after irradiation with 950 cGy radiation, whereby engraftment of the bone marrow graft is promoted, and further, regulatory t cells (Treg) are proliferated to prevent GVHD (Non-Patent Document 1). Leventhal et al. have run a clinical trial of simultaneously performing bone marrow transplantation and kidney transplantation using facilitating cells, and replacing all the blood cells of the recipient with donor cells, thereby successfully engrafting the graft without using immunosuppression and also suppressing GVHD (Non-Patent Document 2). While it is shown that immune tolerance of the transplanted organ can be induced by establishing mixed chimera, intense pretreatment that is almost myeloablative is performed in their experiments, and thus it is difficult to apply this pretreatment to all the organ failure patients. Requirement of treatment of the cells before bone marrow transplantation also makes generalization of this treatment difficult.

S. Strober et al. have shown that graft-versus-host disease (GVHD) is not caused in bone marrow transplantation after fractionated total lymphoid irradiation (TLI), and the state of mixed chimera lasts for a long period, and has reported that the cause thereof is in natural killer T (NKT) cells that become dominant after pretreatment (Non-Patent Document 3). Further, they describe that tolerance of mixed chimera and a transplanted heart is obtained by simultaneously performing bone marrow transplantation and heart transplantation after TLI (Non-Patent Document 4), and then performed HLA-compatible kidney transplantation in human by a similar protocol (Non-Patent Document 5). Radiation irradiation they used in animal experiment is of a considerably high dose of 240 cGy x 10 times although the body was shielded except for the lymphatic organs, the treatment period is also long since it is fractionated irradiation, and further, administration of anti-thymocyte globulin (ATG) is also necessary, but its side-effects cannot be ignored. A protocol using lower dose radiation and not using an agent having cytotoxicity such as ATG as much as possible is more desirable.

DH. Sachs et al. have reported induction of immune tolerance of the transplanted organ by introduction of mixed chimera in 1989 for the first time in the world (Non-Patent Document 6). Thereafter, they have found a protocol for inducing immune tolerance in a less invasive manner by suppressing functions of T cells, by combined use of thymic irradiation and an anti-T cell antibody (Non-Patent Document 7) and combined use of ADDIN EN.CITE ADDIN EN.CITE.DATA or a nonmyeloablative dose (3 Gy) of total body irradiation (TBI), co-stimulation blockade (CB) and an anti-T cell antibody (Non-Patent Documents 8 and 9). They sometimes add CTLA4-Ig to 2 mg of an anti-CD40L antibody as CB, but the anti-CD40 Ligand (L) antibody is known to have a problem of thrombosis (Non-Patent Document 10), and CTLA4-Ig also causes susceptibility to infection, and thus, such CB has issues of side effects problematic in terms of practical use. Therefore, a protocol that avoids the use of such agents, or reduces the amount or does not use such agents as much as possible is necessary. Also, their protocol was effective in the MHC mismatched combination, but they admit that the inductivity of chimera drops in Full allo (Non-Patent Document 11). However, they have succeeded in immune tolerance induction to some extent by attempting clinical applications in monkey and human by applying such protocol (Non-Patent Document 12). In these experiments, involvement of NKT cells is not examined. However, based on the report that NKT cells are important for induction of thymic chimera (Non-Patent Document 4), and the report that NKT cells are essential for immune tolerance induction by co-stimulation blockade (Non-Patent Document 5), it can be assumed that NKT cells play an important role also in the system of Sachs et al. It can be assumed that T cells are required to generate the state advantageous for NKT cells for the NKT cells to work in immune tolerance, also based on the report of xenogeneic islet transplantation of Ikehara et al. (Non-Patent Document 6). Based on these reports, the possibility of inducing mixed chimera by activation of NKT cells is suggested.

On the other hand, Nierlich et al. describe that activation of NKT cells rather inhibitorily worked on the induction of immune tolerance by aqua liquid α-galactosylceramide (hereinafter, may be abbreviated as "α-GalCer") in a CB-based mixed chimera model (Non-Patent Document 7), and Seino et al. disclose that aqua liquid α-GalCer showed only a small effect of extending engraftment in heart transplantation (Non-Patent Document 8). It is considered that such phenomena are thought to be caused by the fact that NKT also plays a role of activating immunity while playing a role of controlling immunity. The detailed mechanism how NKT cells switch these two opposite actions has not been made clear. It is known that a plurality of costimulation systems are also present for NKT cells similarly to general T cells (Non-Patent Document 13), and it is known that the anti-CD40L antibody and CTLA4-Ig stated above suppress activation of immunity by NKT (Non-Patent Document 14). However, there is still no report in which control of transplantation immunity is achieved by combined use of activation of NKT cells and CB.

There has also been an attempt to artificially control transplantation immunity by using a derivative of α-GalCer, and application to transplantation has also been reported (Non-Patent Document 15). However, its effect is limited, and the survival rate of heart transplantation of mice was only slightly improved by using the derivative of α-GalCer in combination with rapamycin that is an immunosuppressant.

The group including the present inventors has reported that liposomal α-GalCer is presented to iNKT cells via CD1d of antigen-presenting cells, whereby the action relating to immune control is high, as compared to aqua liquid α-GalCer (Non-Patent Document 16), and that when liposomal α-GalCer is administered in myeloablative bone marrow transplantation, GVHD is suppressed by proliferation of Treg even if Full chimera is formed (Non-Patent Document 11). Furthermore, it is shown that liposome containing KRN7000 as α-GalCer has an induction action of IL-10 producing T cells and an inhibitory action on IgE antibody production that are not exhibited only by aqua liquid α-GalCer, and is also useful as a preventive or therapeutic agent for allergic diseases, autoimmune diseases and GVHD (Patent Document 17 and Patent Document 1).

### PRIOR ART DOCUMENTS

### PATENT DOCUMENT

Patent Document 1: WO 2005/120574

### NON-PATENT DOCUMENTS

Non-Patent Document 1: Cardenas PA, Huang Y, Ildstad ST. The role of pDC, recipient T(reg) and donor T(reg) in HSC engraftment: Mechanisms of facilitation. Chimerism 2011; 2(3): 65.
Non-Patent Document 2: Leventhal J, Abecassis M, Miller J, et al. Chimerism and tolerance without GVHD or engraftment syndrome in HLA-mismatched combined kidney and hematopoietic stem cell transplantation. Sci Transl Med 2012; 4(124): 124ra28.
Non-Patent Document 3: Lan F, Zeng D, Higuchi M, Huie P, Higgins JP, Strober S. Predominance of NK1.1+TCR alpha beta+ or DX5+TCR alpha beta+ T cells in mice conditioned with fractionated lymphoid irradiation protects against graft-versus-host disease: "natural suppressor" cells. J Immunol 2001; 167(4): 2087.
Non-Patent Document 4: Higuchi M, Zeng D, Shizuru J, et al. Immune tolerance to combined organ and bone marrow transplants after fractionated lymphoid irradiation involves regulatory NK T cells and clonal deletion. J Immunol 2002; 169(10): 5564.
Non-Patent Document 5: Scandling JD, Busque S, Dejbakhsh-Jones S, et al. Tolerance and chimerism after renal and hematopoietic-cell transplantation. N Engl J Med 2008; 358(4): 362.
Non-Patent Document 6: Sharabi Y, Sachs DH. Mixed chimerism and permanent specific transplantation tolerance induced by a nonlethal preparative regimen. J Exp Med 1989; 169(2): 493.
Non-Patent Document 7: Sykes M, Szot GL, Swenson KA, Pearson DA. Induction of high levels of allogeneic hematopoietic reconstitution and donor-specific tolerance without myelosuppressive conditioning. Nat Med 1997; 3(7): 783.
Non-Patent Document 8: Ito H, Kurtz J, Shaffer J, Sykes M. CD4 T cell-mediated alloresistance to fully MHC-mismatched allogeneic bone marrow engraftment is dependent on CD40-CD40 ligand interactions, and lasting T cell tolerance is induced by bone marrow transplantation with initial blockade of this pathway. J Immunol 2001; 166(5): 2970.
Non-Patent Document 9: Takeuchi Y, Ito H, Kurtz J, Wekerle T, Ho L, Sykes M. Earlier Low-Dose TBI or DST Overcomes CD8+ T-Cell-Mediated Alloresistance to Allogeneic Marrow in Recipients of Anti-CD40L. American Journal of Transplantation 2004; 4(1): 31.
Non-Patent Document 10: T. Kawai et. al., Nat. Medi. 6: 114, 2000
Non-Patent Document 11: Bigenzahn S, Blaha P, Koporc Z, et al. The role of non-deletional tolerance mechanisms in a murine model of mixed chimerism with costimulation blockade. Am J Transplant 2005; 5(6): 1237.
Non-Patent Document 12: Sykes M. Mechanisms of transplantation tolerance in animals and humans. Transplantation 2009; 87 (9 Suppl): S67.
Non-Patent Document 13: van den Heuvel MJ, Garg N, Van Kaer L, Haeryfar SMM. NKT cell costimulation: experimental progress and therapeutic promise. Trends in Molecular Medicine 2011; 17(2): 65.
Non-Patent Document 14: Hayakawa Y, Takeda K, Yagita H, Van Kaer L, Saiki I, Okumura K. Differential regulation of Th1 and Th2 functions of NKT cells by CD28 and CD40 costimulatory pathways. J Immunol 2001; 166(10): 6012.
Non-Patent Document 15: Haeryfar SMM, Lan Z, Leon-Ponte M, et al. Prolongation of Cardiac Allograft Survival by Rapamycin and the Invariant Natural Killer T Cell Glycolipid Agonist OCH. Transplantation 2008; 86(3): 460.
Non-Patent Document 16: Yasuyuki Ishii, Risa Nozawa et al. Alpha-galactosylceramide-driven immunotherapy for allergy. Frontiers in Bioscience 13,6214-6228
Non-Patent Document 17: Omar Duramad, Amy Laysang, Jun Li, Yasuyuki Ishii, Reiko Namikawa. Pharmacologic Expansion of Donor-Derived, Naturally Occurring CD41Foxp31 Regulatory T Cells Reduces Acute Graft-versus-Host Disease Lethality Without Abrogating the Graft-versus-Leukemia Effect in Murine Models. Biol Blood Marrow Transplant. 1-15 (2011)

### SUMMARY OF THE INVENTION

### PROBLEMS TO BE SOLVED BY THE INVENTION

An object of the present invention is, in a therapy in which donor hematopoietic cells are transplanted into a recipient to induce mixed himera, in order to induce immune tolerance in the recipient with respect to donor cells, tissue, or organ, to create a safe and practical immune tolerance-inducing agent that can induce appropriate immune tolerance on the transplanted organ, and a usage thereof.

### MEANS FOR SOLVING THE PROBLEMS

The present inventors have intensively studied to solve the above problems, and have found that a hematopoietic cell chimera by transplantation of donor hematopoietic cells can be induced in the recipient by using liposome containing α-GalCer and a substance inhibiting costimulatory pathway in combination, whereby it becomes possible to induce immune tolerance in the recipient with respect to donor cells, tissue, or organ. The present invention has been accomplished by further studies based on the above knowledge.

More specifically, the present invention provides an immune tolerance-inducing agent, a hematopoietic cell chimeria-inducing agent, and the like, of the aspects described below.
Item 1. An immune tolerance-inducing agent used in a therapy in which donor hematopoietic cells are transplanted into a recipient in order to induce immune tolerance in the recipient with respect to donor cells, tissue, or organ, containing liposome containing α-galactosylceramide and a substance inhibiting costimulatory pathway.
Item 2. The immune tolerance-inducing agent according to item 1, wherein the costimulatory pathway is CD40/CD40L costimulatory pathway.
Item 3. The immune tolerance-inducing agent according to item 1 or 2, wherein the substance inhibiting costimulatory pathway is an anti-CD40 antibody or an anti-CD40L antibody.
Item 4. The immune tolerance-inducing agent according to any one of items 1 to 3, wherein the donor hematopoietic cells to be transplanted are hematopoietic cells derived from bone marrow, cord blood, or peripheral blood.
Item 5. The immune tolerance-inducing agent according to any one of items 1 to 3, wherein the donor hematopoietic cells to be transplanted are bone marrow.
Item 6. The immune tolerance-inducing agent according to any one of items 1 to 5, wherein the donor cells, tissue or organ are a part or the whole of the heart, lung, liver, small intestine, skin, kidney or pancreas derived from the donor.
Item 7. The immune tolerance-inducing agent according to any one of items 1 to 6, wherein the liposome containing α-galactosylceramide and the substance inhibiting costimulatory pathway are contained in one preparation.
Item 8. The immune tolerance-inducing agent according to any one of items 1 to 6, which is of a two-agent form including a preparation containing the liposome containing α-galactosylceramide and a preparation containing the substance inhibiting costimulatory pathway.
Item 9. A method for inducing immune tolerance in a recipient with respect to donor cells, tissue, or organ, including administering liposome containing α-galactosylceramide and a substance inhibiting costimulatory pathway to the recipient into which donor hematopoietic cells were transplanted or the recipient into which donor hematopoietic cells are to be transplanted.
Item 10. Use of liposome containing α-galactosylceramide and a substance inhibiting costimulatory pathway for producing an immune tolerance-inducing agent used in a therapy in which donor hematopoietic cells are transplanted into a recipient in order to induce immune tolerance in the recipient with respect to donor cells, tissue, or organ.
Item 11. A hematopoietic cell chimeria-inducing agent, containing liposome containing α-galactosylceramide and a substance inhibiting costimulatory pathway.
Item 12. The hematopoietic cell chimeria-inducing agent according to item 11, which is administered to a recipient into which donor hematopoietic cells were transplanted or a recipient into which donor hematopoietic cells are to be transplanted.
Item 13. The hematopoietic cell chimeria-inducing agent according to item 12, wherein the donor hematopoietic cells are hematopoietic cells derived from bone marrow, cord blood, or peripheral blood.
Item 14. The hematopoietic cell chimeria-inducing agent according to any one of items 11 to 13, wherein the costimulatory pathway is CD40/CD40L costimulatory pathway.
Item 15. The hematopoietic cell chimeria-inducing agent according to any one of items 11 to 14, wherein the substance inhibiting costimulatory pathway is an anti-CD40 antibody or an anti-CD40L antibody.
Item 16. A method for inducing formation of a hematopoietic cell chimera in the body of a recipient, including administering liposome containing α-galactosylceramide and a substance inhibiting costimulatory pathway to the recipient into which donor hematopoietic cells were transplanted or the recipient into which donor hematopoietic cells are to be transplanted.
Item 17. Use of liposome containing α-galactosylceramide and a substance inhibiting costimulatory pathway for producing a hematopoietic cell chimeria-inducing agent.

### ADVANTAGES OF THE INVENTION

According to the immune tolerance-inducing agent of the present invention, in a therapy in which donor hematopoietic cells are transplanted into a recipient in order to induce immune tolerance in the recipient with respect to donor cells, tissue, or organ, a hematopoietic cell chimera is prepared in the body of the recipient and can be stably maintained, and immune tolerance in transplantation immunity can be effectively induced. In addition, the immune tolerance-inducing agent of the present invention can facilitate proliferation of regulatory T cells in the body of a recipient, and it is also considered to contribute to effective induction of immune tolerance.

Furthermore, in transplantation treatment, pretreatment focusing on radiation irradiation is conventionally essential. However, when the immune tolerance-inducing agent of the present invention is used, immune tolerance can be effectively induced, and thus there is also a possibility that such pretreatment is carried out under mild conditions so that the burden on the recipient can be reduced.

In addition, there is a possibility that immune tolerance can be efficiently induced also in intractable autoimmune diseases such as systemic sclerosis, systemic lupus erythematosus, rheumatoid arthritis, dermatomyositis, multiple sclerosis, Crohn's disease, and autoimmune cytopenia that are sometimes treated by transplantation of hematopoietic cells.

In addition, since the hematopoietic cell chimeria-inducing agent of the present invention can efficiently prepare a hematopoietic cell chimera in the body of a recipient, not only it is used for the purpose of inducing tolerance in transplantation immunity, but also preparation of hematopoietic cell chimeric nonhuman animal usable as an experimental model animal becomes possible.

### BRIEF DESCRIPTION OF THE DRAWINGS

Fig. 1 shows a result of dyeing MHC class I antigen with both recipient type (H2K^{d}) and donor type (H2K^{b}), for peripheral blood monocytes and spleen cells of a recipient mouse in Example 1.
Fig. 2 shows a result of evaluating engraftment of donor cells in peripheral blood, spleen, lymph node, and bone marrow, for a TBI+anti-CD40L+KRN7000-containing liposome group 200 days after bone marrow transplantation in Example 1.
Fig. 3 shows a result of evaluating the ratio of donor cells in the thymus (graph on the right), and expression of donor MHC class I antigen in the blood cell, for a TBI+anti-CD40L+KRN7000-containing liposome group 200 days after bone marrow transplantation in Example 1.
Fig. 4A shows a result of measuring weight change after bone marrow transplantation of a TBI+anti-CD40L+KRN7000-containing liposome group and a TBI+anti-CD40L group in Example 1.
Fig. 4B shows a result of observing that no graft rejection is recognized in the tissue images of the small intestine (photographs on the left) and the liver (photographs on the right) of a TBI+anti-CD40L+KRN7000-containing liposome group 100 days after transplantation in Example 1.
Fig. 5 shows a result of evaluating expression of donor MHC class I antigen, for CD24(+)CD4(-)CD8(-) T cells recognized in a TBI+anti-CD40L+KRN7000-containing liposome group in Example 1.
Fig. 6 shows a result of performing heart transplantation of a TBI+anti-CD40L+KRN7000-containing liposome group and a TBI+anti-CD40L group and observing engraftment of the heart graft in Example 2.
Fig. 7 shows a result of observing that no rejection is recognized in the tissue image of a TBI+anti-CD40L+KRN7000-containing liposome group 100 days after heart transplantation in Example 2.
Fig. 8 shows a result of measuring serum IL-2 for recipient mice of each group 2, 24 and 48 hours after bone marrow transplantation in Example 3.
Fig. 9 shows a result of measuring serum IL-4 for recipient mice of each group 2, 24 and 48 hours after bone marrow transplantation in Example 3.
Fig. 10 shows a result of measuring serum IL-10 for recipient mice of each group 2, 24 and 48 hours after bone marrow transplantation in Example 3.
Fig. 11 shows a result of measuring serum IL-12 for recipient mice of each group 2, 24 and 48 hours after bone marrow transplantation in Example 3.
Fig. 12 shows a result of measuring serum IFN-γ for recipient mice of each group 2, 24 and 48 hours after bone marrow transplantation in Example 3.
Fig. 13A shows a result of measuring the ratio and activation of Treg in the spleen cells for recipient mice of each group in Example 3.
Fig. 13B shows a result of measuring the ratio and activation of Treg in the spleen cells for recipient mice of each group in Example 3.
Fig. 13C shows a result of measuring the ratio and activation of Treg in the spleen cells for recipient mice of each group in Example 3.
Fig. 13D shows a result of measuring the ratio and activation of Treg in the spleen cells for recipient mice of each group in Example 3.
Fig. 14 shows that Treg (and activated Treg: aTreg) was deleted by administering an anti-CD25 antibody in Example 3.

### EMBODIMENTS OF THE INVENTION

### 1. Immune tolerance-inducing agent

The immune tolerance-inducing agent of the present invention is used in a therapy in which donor hematopoietic cells are transplanted into a recipient in order to induce immune tolerance in the recipient with respect to donor cells, tissue, or organ, and contains liposome containing α-galactosylceramide and a substance inhibiting costimulatory pathway. Hereinbelow, the present invention will be described in detail.

The α-galactosylceramide (α-GalCer) used in the present invention is a glycosphingolipid in which galactose and ceramide are bound in α-configuration, and specific examples thereof include those disclosed in WO94/09020, WO94/02168, WO94/24142, WO98/44928, Science, 278, pp. 1626-1629, 1997, and the like. In the present invention, from the viewpoint of further improving the effect of inducing immune tolerance, so-called KRN7000, i.e., (2S,3S,4R)-1-O-(α-D-galactopyranosyl)-2-hexacosanoylamino-1,3,4-octadecanetriol is preferably used as α-GalCer.

In the present invention, α-GalCer is used in the state of being included in liposome. More specifically, liposome including α-GalCer is used in the present invention. Since the ceramide moiety of α-GalCer shows lipophilicity, α-GalCer included in liposome usually has a structure in which α-GalCer is localized in the lipid bilayer membrane of liposome. Hereinafter, the liposome including α-GalCer may be referred to as liposomal α-GalCer.

Preferable examples of the liposomal α-GalCer used in the present invention can include KRN7000-containing liposome produced using KRN7000 as α-GalCer. In addition, particularly preferable examples can include RGI-2001 that is a KRN7000-containing liposome preparation (manufactured by REGiMMUNE Co., Ltd.), prepared in the same manner as in the method described in Biol Blood Marrow Transplant. 1-15 (2011).

When the α-GalCer included in liposome is used in the present invention, the ratio of constituent lipid of liposome to α-GalCer can be properly set by a person skilled in the art depending on the intended use, and is not particularly limited. For example, α-GalCer is contained in an amount of 0.05 to 100 parts by weight, preferably 0.5 to 20 parts by weight per the total amount of 100 parts by weight of the constituent lipid of liposome.

The lipid used in the liposomal α-GalCer (constituent lipid of liposome) is not particularly limited as long as the lipid can form a double membrane structure. Specific examples of the liposome constituent lipid include diacylphosphatidylcholines such as dipalmitoylphosphatidylcholine (DPPC), dioleoylphosphatidylcholine (DOPC), dimyristoylphosphatidylcholine (DMPC), and disteroylphosphatidylcholine (DSPC); diacylphosphatidylglycerols such as dipalmitoylphosphatidylglycerol (DPPG), dioleoylphosphatidylglycerol (DOPG), dimyristoylphosphatidylglycerol (DMPG), and disteroylphosphatidylglycerol (DSPG); sterols such as cholesterol, 3β-[N-(dimethylaminoethane)carbamoyl] cholesterol (DC-Chol), N-(trimethylammonioethyl)carbamoyl cholesterol (TC-Chol), tocopherol, cholesterol succinate, lanosterol, dihydrolanosterol, desmosterol, dihydrocholesterol, zymosterol, ergosterol, stigmasterol, sitosterol, campesterol, and brassicasterol; phosphatidylethanolamines such as dioleoylphosphatidylethanolamine (DOPE), disteroylphosphatidylethanolamine (DSPE), and polyethylene glycol-phosphatidylethanolamine (PEG-PE); phosphatidines such as dimyristoylphosphatidic acid; gangliosides such as ganglioside GM1; polyethylene glycol fatty acid esters such as polyethylene glycol palmitate and polyethylene glycol myristate, and the like.

As the liposome constituent lipid, one kind may be used alone, but it is preferable to use two or more kinds in combination. Preferable examples of the combination of the liposome constituent lipid include combinations of diacylphosphatidylcholines, diacylphosphatidylglycerols and sterols, and combinations of diacylphosphatidylcholines and sterols; and further preferable examples include a combination of DPPC, DOPC, DPPG and cholesterol, and combinations of DOPC, cholesterol and/or DC-Chol.

When two or more kinds of liposome constituent lipids are used in combination, the compounding ratio of each lipid is properly set in consideration of the size, flowability and the like that are required for liposome. For example, when the combination of diacylphosphatidylcholines, diacylphosphatidylglycerols and sterols is adopted, diacylphosphatidylcholines : diacylphosphatidylglycerols : sterols is 1 : 0.125 to 0.75 : 0.125 to 1, preferably 1 : 0.14 to 0.4 : 0.14 to 0.6 by molar ratio. Also, for example, when the combination of DPPC, DOPC, DPPG and cholesterol is adopted, DPPC : DOPC : DPPG : cholesterol is 1 : 0.16 to 1.65 : 0.16 to 1.0 : 0.16 to 1.3, preferably 1 : 0.4 to 0.75 : 0.2 to 0.5 : 0.3 to 0.75 by molar ratio. Moreover, for example, when the combination of diacylphosphatidylcholines (preferably DOPC) and sterols (preferably cholesterol and/or DC-Chol) is adopted, diacylphosphatidylcholines : sterols is 1 : 0.05 to 4, preferably 1 : 0.1 to 1 by molar ratio.

When α-GalCer is liposomized, liposome may contain a cationic compound such as stearylamine and oleylamine; an anionic compound such as dicetyl phosphate; and a membrane protein, as necessary, and the compounding ratio thereof can be properly set.

When α-GalCer is liposomized, the size of liposome is not particularly limited, and the average particle size is usually 5 to 1000 nm, preferably 100 to 400 nm. The average particle size of liposome is measured by dynamic light scattering. Also, the structure of liposome is not particularly limited, and may be any of MLV (multilamellar vesicles), DRV (dehydration-rehydration vesicles), LUV (large umilamellar vesicles) or SUV (small unilamellar vesicles).

When α-GalCer is liposomized, examples of the solution to be contained in liposome include pharmaceutically acceptable aqueous carriers such as water, a buffer, and physiological saline.

The liposomal α-GalCer is prepared using a known liposome production method such as hydration, ultrasonic treatment, ethanol injection, ether injection, reverse phase evaporation, a surfactant method, or a freezing and thawing method. Also, liposome is allowed to pass through a filter with a predetermined pore size, whereby the particle size distribution of liposome can be adjusted. Moreover, according to a known method, conversion from MLV to a unilamellar liposome and conversion from a unilamellar liposome to MLV can also be performed.

The "substance inhibiting costimulatory pathway" (hereinafter, sometimes simply referred to as inhibitory substance) used in the present invention is a substance inhibiting costimulatory pathway between antigen-presenting cells and T cells in order to activate the T cells. The inhibitory substance is not particularly limited as long as the substance can inhibit costimulatory pathway, and a low-molecular compound, an antibody, a protein, a nucleic acid (aptamer, antisense molecule, siRNA, etc.) and the like can be used.

Specific examples of the costimulatory pathway include CD86/CD28 costimulatory pathway, CD80/CD28 costimulatory pathway, CD40/CD40L costimulatory pathway, OX40/OX40L costimulatory pathway, COS/ICOSL costimulatory pathway, and the like. The inhibitory substance used in the present invention may be one inhibiting the function of either one of costimulatory molecule and auxiliary stimulatory molecule constituting the costimulatory pathway, or may be one inhibiting the functions of both of them. Specific examples of the inhibitory substance include low-molecular compounds that can inhibit the function to at least one kind of CD86, CD28, CD80, CD40 and CD40L; antibodies bound to at least one kind of them; aptamers, antisense molecules or siRNAs to at least one kind of them, and the like.

Preferred examples of the costimulatory pathway as an inhibitory object of the inhibitory substance include CD40/CD40L costimulatory pathway. More specifically, preferred examples of the inhibitory substance include low-molecular compounds that can inhibit the function to CD40 and/or CD40L; antibodies bound to CD40 and/or CD40L; aptamers, antisense molecules or siRNAs to CD40 and/or CD40L, and the like. Among them, further preferred examples of the inhibitory substance include an anti-CD40 antibody and an anti-CD40L antibody. The substance inhibiting CD40/CD40L costimulatory pathway and the liposomal α-GalCer are simultaneously used, thereby more efficiently inducing production of a hematopoietic cell chimera performed by transplantation of donor hematopoietic cells in the recipient, and consequently it becomes possible to further effectively induce immune tolerance in the recipient with respect to donor cells, tissue, or organ.

In the immune tolerance-inducing agent of the present invention, the ratio of the liposome containing α-GalCer to the substance inhibiting costimulatory pathway is not particularly limited, but the liposome containing α-GalCer is contained in an amount of 0.001 to 5000 parts by weight, preferably 0.05 to 100 parts by weight, further preferably 0.25 to 20 parts by weight, expressed in terms of weight of α-GalCer, based on the substance inhibiting costimulatory pathway.

In the immune tolerance-inducing agent of the present invention, the liposome containing α-GalCer and the substance inhibiting costimulatory pathway may be contained in one preparation, or contained each in two different preparations. More specifically, one aspect of the immune tolerance-inducing agent of the present invention is a preparation for immune tolerance induction (single package type) containing liposome containing α-GalCer and a substance inhibiting costimulatory pathway, and another aspect thereof is a kit for immune tolerance induction (two package type) including a preparation containing liposome containing α-GalCer and a preparation containing a substance inhibiting costimulatory pathway.

The dosage form of the immune tolerance-inducing agent of the present invention can be properly set depending on the administration form, and may be any form such as liquid, powder, granule, tablet, or capsule. Also, when the immune tolerance-inducing agent of the present invention is of a two package type, the preparation containing liposome containing α-GalCer and the preparation containing a substance inhibiting costimulatory pathway may be in the same dosage form or may be two different preparations.

The immune tolerance-inducing agent of the present invention contains a pharmaceutically acceptable carrier, as necessary, other than the liposome containing α-GalCer and the substance inhibiting costimulatory pathway, and is prepared to a desired dosage form. Examples of the pharmaceutically acceptable carrier include aqueous carriers such as distilled water, physiological saline, phosphate buffer, citrate buffer, and acetate buffer; saccharides such as sucrose, fructose, saccharose, glucose, lactose, mannitol, and sorbitol; polyhydric alcohols such as glycerin, propylene glycol, and butylene glycol; surfactants such as nonionic surfactants, cationic surfactants, negative surfactants, and amphoteric surfactants; cellulose derivatives such as hydroxypropyl methylcellulose, hydroxypropyl cellulose, methyl cellulose, ethyl cellulose, hydroxypropyl methylcellulose phthalate, hydroxypropyl methylcellulose acetate succinate, and carboxymethyl ethyl cellulose; antioxidants; pH adjusting agents, and the like.

The immune tolerance-inducing agent of the present invention is administered to a patient (recipient) who is subjected to transplantation of donor cells, tissue, or organ, and is used in a therapy in which donor hematopoietic cells are transplanted into a recipient in order to induce immune tolerance in the recipient with respect to transplanted cells, tissue, or organ. More specifically, the immune tolerance-inducing agent of the present invention is administered, in the recipient in need of the transplantation of cells, tissue, or organ, when donor hematopoietic cells are transplanted into the recipient in order to induce immune tolerance in the recipient with respect to donor cells, tissue, or organ, prior to or concurrently with the transplantation.

The donor cells, tissue, or organ to be transplanted into a patient are not particularly limited, and examples include a part or the whole of the heart, lung, liver, small intestine, skin, kidney or pancreas derived from the donor.

The "donor hematopoietic cell" in the present invention is a hematopoietic cell on the donor side to be transplanted into a recipient, and refers to a stem cell differentiable into a blood cell. The originating tissue of the hematopoietic cell to be transplanted into a recipient is not particularly limited, and for example, may be bone marrow, cord blood, or peripheral blood itself, and may be a hematopoietic cell derived from bone marrow, cord blood, or peripheral blood. Preferred examples of the hematopoietic cell to be transplanted into a recipient include hematopoietic cells derived from bone marrow, cord blood, or peripheral blood. Any transplant amount of donor hematopoietic cells is acceptable as long as it is an effective amount that can induce immune tolerance. The transplant amount may be properly set depending on the symptom, age and the like of the recipient, and is usually about 1 x 10⁶ to 3 x 10⁸ cells/kg.

The immune tolerance-inducing agent of the present invention may be administered between 72 hours before transplantation and 72 hours after transplantation of the donor hematopoietic cells, and is preferably administered concurrently with the transplantation of the donor hematopoietic cells or within 24 hours after transplantation.

When the immune tolerance-inducing agent of the present invention is of a two package type, the order of administration of the preparation containing liposome containing α-GalCer and the preparation containing a substance inhibiting costimulatory pathway is not particularly limited, and they may be administered simultaneously or in an arbitrary order.

The administration form of the immune tolerance-inducing agent of the present invention may be either of parenteral administration and oral administration. Specific examples thereof include intravenous administration, intramuscular administration, intraperitoneal administration, subcutaneous administration, intraarticular administration, mucosal administration, and the like. Among these administration forms, from the viewpoint of further improving induction of immune tolerance, parenteral administration, particularly intravenous administration is preferable.

Examples of the subject of administration of the immune tolerance-inducing agent of the present invention include mammals such as human, monkey, mouse, rat, dog, rabbit, cat, cow, horse, and goat; and birds such as chicken and ostrich. The subject is preferably a human.

The administration amount of the immune tolerance-inducing agent of the present invention may be any amount as long as it is an effective amount that can induce immune tolerance, and may be properly set depending on the symptom, age and the like of the recipient. The amount may be about 1 to 100 µg/kg expressed in terms of the amount of α-GalCer to be administered.

Usually, transplantation of the hematopoietic cells is performed on a patient having a disease which makes it difficult to form healthy blood, but in the present invention, it is performed for preparing a hematopoietic cell chimera for inducing immune tolerance in the recipient with respect to donor cells, tissue, or organ. More specifically, transplantation of the donor hematopoietic cells and administration of the immune tolerance-inducing agent of the present invention are performed to a recipient, whereby a hematopoietic cell chimera is efficiently prepared in the body of a recipient, and the hematopoietic cell chimera can induce immune tolerance with respect to transplanted cells, tissue, or organ.

Also, in normal transplantation treatment, pretreatment such as systemic radiation irradiation to the recipient is essential. Since this pretreatment is performed for the purpose of killing hematopoietic cells of the recipient, a high radiation dose of about 10 to 50 Gy is necessary, and a large burden is imposed on the recipient.

However, when immune tolerance is induced to prepare a hematopoietic cell chimera as a prerequisite for transplantation treatment using the immune tolerance-inducing agent of the present invention, immune tolerance can be efficiently induced even under nonmyeloablative conditions of hematopoietic cells, in which radiation irradiation in the pretreatment is mild such as about 1 to 3 Gy, as shown in the examples set forth below. Therefore, the burden on the recipient can be reduced, and thus there is a possibility of expansively applying induction of immune tolerance also to a patient to whom it is not applicable so far, such as a recipient suffering from an intractable autoimmune disease.

The "hematopoietic cell chimera" in the present invention means the state of coexistence of donor hematopoietic cells and recipient hematopoietic cells in the body of a recipient. In the state of a hematopoietic cell chimera, donor-derived antigen-presenting cells remove donor-reactive T-cells in the thymus, and thus specific immune tolerance is established with respect to donor cells, tissue, or organ.

As also described in the section of BACKGROUND ART, in the conventionally known production of a hematopoietic cell chimera, a protocol using not only radiation irradiation but also a substance suppressing functions of T cells (anti-T cell antibody, etc.) and further using plural kinds of substances inhibiting costimulatory pathways in large amounts is adopted. However, in Full allo, a production method suitable for practical use has not been established, such that the inductivity of chimera is lowered, for example.

According to the immune tolerance-inducing agent of the present invention, in the preparation of a hematopoietic cell chimera as a prerequisite for immune tolerance induction, a substance inhibiting costimulatory pathway may be used in a small amount (For example, the amount is 50 mg/kg or less, and when the substance inhibiting costimulatory pathway is an anti-CD40L antibody, the amount is about 25 mg/kg. That is, the amount is 1 mg or less when the substance inhibiting costimulatory pathway is administered to a mouse with a weight of 20 g, and when the substance inhibiting costimulatory pathway is an anti-CD40L antibody, the amount is about 0.5 mg/kg.), and plural kinds are not also necessary. The examples set forth below show that a hematopoietic cell chimera which still stably exists over a long period of time can be induced.

Also, according to the immune tolerance-inducing agent of the present invention, it is also not necessary to prepare ex vivo a cell into which tolerance is easily introduced, such as a facilitating cell.

More specifically, it can be understood that the immune tolerance-inducing agent of the present invention is an immune tolerance-inducing agent that can be put to practical use for the first time in the induction of a hematopoietic cell chimera.

Based on the above, the immune tolerance-inducing agent of the present invention requires only the use of low dose radiation in the induction of a hematopoietic cell chimera, and also goes without using an agent with side effect or cytotoxicity (substance inhibiting costimulatory pathway) as much as possible, and thus is proved to be an immune tolerance-inducing agent that can be put to practical use for the first time. It is not described in any known non-patent document that liposome containing α-galactosylceramide and a substance inhibiting costimulatory pathway are contained as the active ingredient of the immune tolerance-inducing agent having such a remarkable effect, and it is not easily conceivable even to a person skilled in the art.

### 2. Hematopoietic cell chimeria-inducing agent

As described above, when donor hematopoietic cells are transplanted to the recipient, liposome containing α-GalCer and a substance inhibiting costimulatory pathway are administered in combination, thereby promoting the preparation of a hematopoietic cell chimera in the body of a recipient. Therefore, the present invention also further provides a hematopoietic cell chimeria-inducing agent, containing liposome containing α-GalCer and a substance inhibiting costimulatory pathway. The present invention further provides a method for preparing a hematopoietic cell chimeric animal using the hematopoietic cell chimeria-inducing agent.

For the hematopoietic cell chimeria-inducing agent, the components, dosage form, subject of administration, administration method and the like are the same as those in the case of the immune tolerance-inducing agent. In addition, the method for preparing the hematopoietic cell chimeric animal is also as described in the column of the immune tolerance-inducing agent.

### EXAMPLES

Hereinbelow, the present invention will be described in detail with reference to examples. However, the present invention is not limited to the examples.

### Example 1: Bone marrow transplantation and preparation of bone marrow chimera

Male mice C57BL/6NCrSlc (B6, H-2^{b}), BALB/cCrSlc (BALB/c, H-2^{d}), and C3H/HeSlc (C3H, H-2^{k}) of 8 to 12 weeks old were purchased from Japan Slc, Inc. The mice were bred in accordance with animal management guidelines of NIH, under an SPF environment of Institute of Laboratory Animals, Tokyo Women's Medical College.

The recipient BALB/c mouse was irradiated with TBI (total body irradiation) 3Gy (X-ray generator (MBR-1520R-3, Hitachi medical Corp., Tokyo, Japan) 2 to 3 hours before bone marrow transplantation. Subsequently, an anti-CD40L antibody was diluted with PBS (Invitrogen, Grand Island, NY, USA), and 0.5 mg of the anti-CD40L antibody was intraperitoneally administered immediately after bone marrow transplantation. The anti-CD40L antibody was purchased from Bixcell (West Lebanon, NH, USA). Further, KRN7000-containing liposome (containing 10% of KRN7000 expressed in terms of the lipid amount) obtained by the same method as in WO 2005/120574 was diluted with Hanks' Balanced Salt Solution (HBSS; Invitrogen, Grand Island, NY, USA), and intravenously administered from the tail vein at a dose of 0.01 µg to 100 µg/kg, expressed in terms of KRN7000, 30 minutes after bone marrow transplantation.

The donor B6 mouse was euthanatized, then the thighbone and the shinbone were collected, and the bone marrow was washed out from the bone marrow cavity using a 21 gauge needle. The bone marrow cells were suspended in HBSS, passed through 70 µm nylon mesh, and then centrifuged at 1800 rpm for 5 minutes. The resulting bone marrow cells were reacted with 2 ml of ACK Lysing Buffer (Lonza MD, USA) at room temperature for 10 minutes, then resuspended in HBSS, the number of cells was counted and adjusted to 2 x 10⁷ bone marrow cells per mouse, and the suspension was intravenously administered from the tail vein of the recipient Balb/c mouse (TBI+anti-CD40L+KRN7000-containing liposome group).

From 28 to 200 days after bone marrow transplantation, peripheral blood was collected from the recipient BALB/c mouse, and reacted with ACK Lysing Buffer (Lonza, MD, USA) at room temperature for 5 minutes. Then, erythrocytes were crushed. MHC class I antigen of the resulting peripheral blood monocyte PBMC was dyed with both recipient type (PE-conjugated anti-H2K^{d}) and donor type (FITC-conjugated anti-H2K^{b}). Further, the resulting MHC class I antigen was dyed using anti-TCRβ, anti-B220, anti-Gr1, and anti-MAC1 (BD Bioscience, CA, USA), in order to distinguish fractionation of blood cells. For dyeing, 1 x 10⁶ cells were suspended in 100 µl of Cell Staining Buffer (CSB: PBS to which 5% Fetal Bovine Serum (Invitrogen, Grand Island, NY, USA) and 0.5 g of Sodium azide (Sigma aldrich, St. Louis, MO, USA) are added), each 10 µL of various antibodies were added, and the mixture was reacted at 4°C for 30 minutes. The reaction mixture was determined by BD FACSCant II (BD Bioscience) and analyzed using FACSDiva (BD Bioscience).

Also, for comparison, the tests were performed in the same manner as described above, also for the case of performing bone marrow transplantation only by radiation irradiation (TBI group), the case of performing radiation irradiation and KRN7000-containing liposome administration (TBI+KRN7000-containing liposome group), and the case of performing radiation irradiation and anti-CD40L antibody administration (TBI+anti-CD40L group). This test was carried out with n = 25 for the TBI+anti-CD40L+KRN7000-containing liposome group, n = 8 for the TBI group, n = 8 for the TBI+KRN7000-containing liposome group, and n = 22 for the TBI+anti-CD40L group.

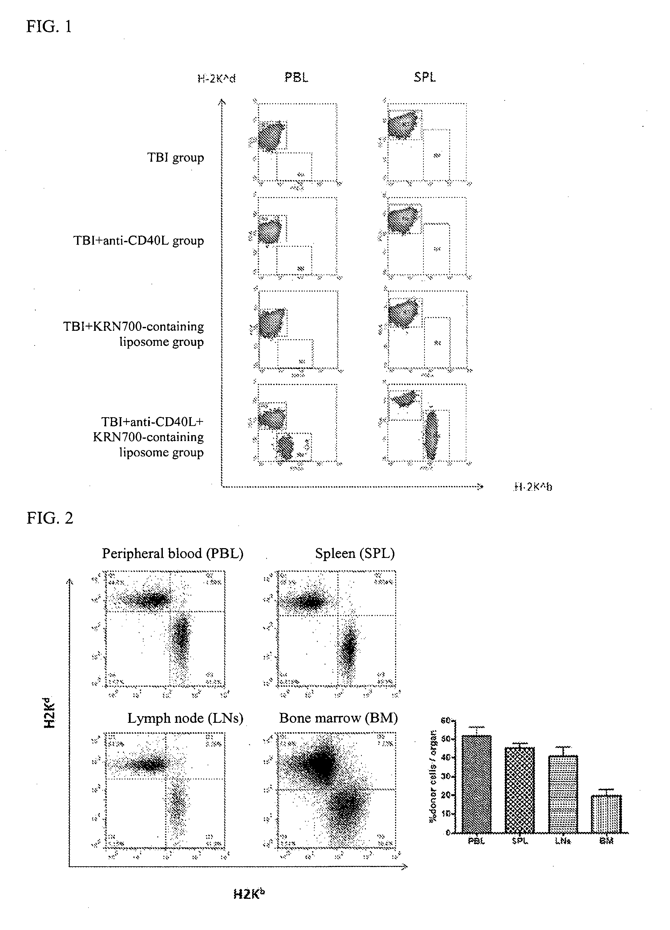

The obtained results are shown in Table 1. As is clear from Table 1, in the TBI group, the TBI+KRN7000-containing liposome group, and the TBI+anti-CD40L group, donor cells were not recognized in the peripheral blood 28 days after bone marrow transplantation, and the graft was rejected. On the other hand, in the TBI+anti-CD40L+KRN7000-containing liposome group, the presence of donor cells was confirmed in the peripheral blood (PBL) of 92.0% of mice (refer to Fig. 1). Also, in the mice in the TBI+anti-CD40L+KRN7000-containing liposome group, the donor cells once engrafted did not disappear after that, and its presence was confirmed even on the 200th after transplantation.

**[Table 1]**

| | Incidence of bone marrow chimera | Ratio of donor cells in peripheral blood lymphocytes (%) | | | |
|---|---|---|---|---|---|
| | | After 28 days | After 60 days | After 100 days | After 200 days |
| TBI group | 0/8(0.0%) | <5 | ND | ND | ND |
| TBI+KRN7000-containing liposome group | 0/8 (0.0%) | <5 | ND | ND | ND |
| TBI+anti-CD40L group | 0/22 (0.0%) | <5 | ND | ND | ND |
| TBI+anti-CD40L+KRN7000-containing liposome group | 23/25 (92.0%) | 51.1 ± 17.6 | 50.8 ± 20.8 | 46.5 ± 23.3 | 41.1 ± 20.2 |

Also, when the administration amount of KRN7000-containing liposome was changed by the same protocol as in the TBI+anti-CD40L+KRN7000-containing liposome group, it was confirmed that engraftment of donor cells 14 days after bone marrow transplantation was dependent on the dose of KRN7000-containing liposome, as shown in Table 2.

**[Table 2]**

| | Administration amount of KRN7000-containing liposome (µg/kg) | | | | | |
|---|---|---|---|---|---|---|
| | 0 | 0.01 | 0.1 | 1.0 | 10 | 100 |
| Ratio of donor cells after 14 days (%) | 0.0 | 0.6 ± 1.1 | 1.9 ± 2.3 | 19.3 ± 23.4 | 43.9 ± 9.9 | 50.0 ± 8.6 |

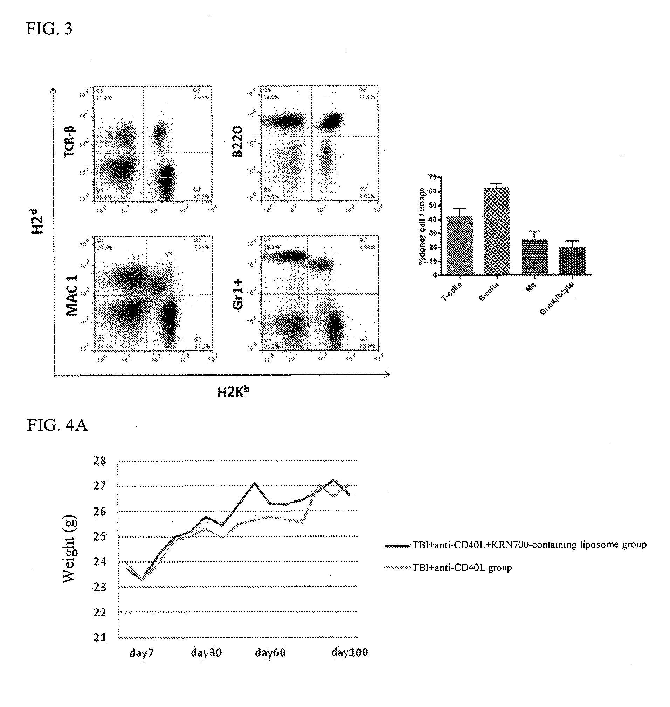

Also in the spleen (SPL), similarly to the peripheral blood, engraftment of donor cells was recognized only in the TBI+anti-CD40L+KRN7000-containing liposome group (refer to Fig. 1). In addition, it was also confirmed that donor cells were engrafted not only in the spleen, but also in the lymph node, bone marrow, and thymus, in the TBI+anti-CD40L+KRN7000-containing liposome group 200 days after bone marrow transplantation (refer to Fig. 2). Moreover, expression of donor MHC class I antigen was recognized in all series of the blood cells, including TCR-β positive cells (T-cells), B220 positive cells (B-cells), MAC-1 positive macrophages (Mq), and Gr-1 positive granulocytes (Granulocyte), in the TBI+anti-CD40L+KRN7000-containing liposome group 200 days after bone marrow transplantation (refer to Fig. 3).

Based on the above results, it was shown that concurrent administration of an anti-CD40L antibody and KRN7000-containing liposome after bone marrow transplantation enables preparation of a bone marrow chimera that is stable for a long period.

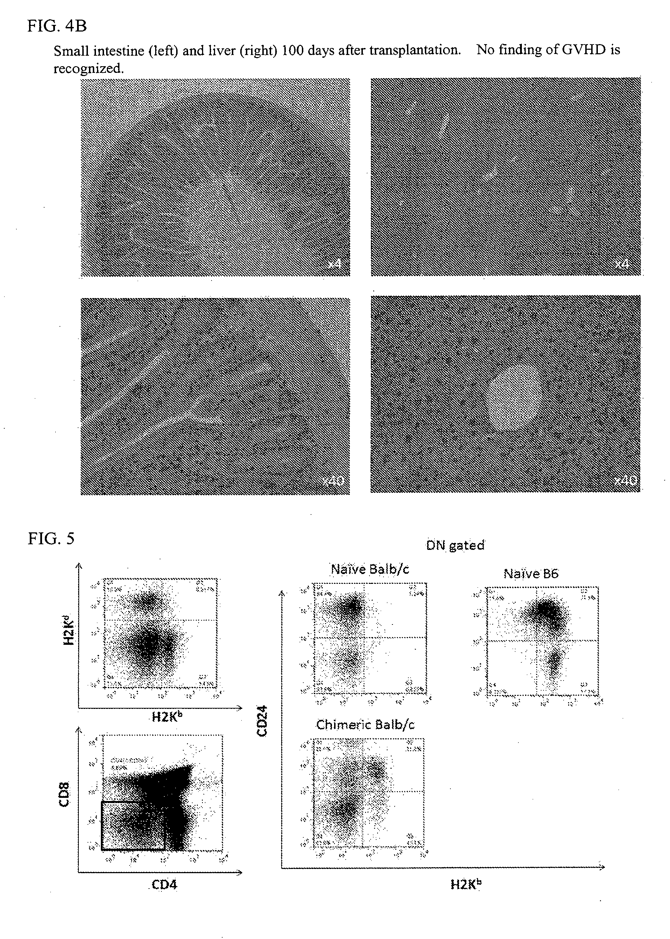

As described above, by the immune tolerance-inducing agent of the present invention, donor cells are engrafted in each tissue without being rejected. Something that deserves special mention is that, as shown in Fig. 4A, weight reduction or digestive organ symptoms that made us suspect GVHD were not recognized at all in the recipient mice to which the immune tolerance-inducing agent of the present invention was administered. Usually, onset of GVHD is concerned if a donor T cell is recognized after bone marrow transplantation, but when the immune tolerance-inducing agent of the present invention is used, no finding that the donor T cell attacks a host is recognized (Fig. 4B). Actually, also in vitro, it is confirmed that, while the reactivity to 3rd party of T cells extracted from a chimeric mouse is maintained, the reactivity to the recipient and the donor is lowered. More specifically, it is shown that T cells that specifically react with a donor are suppressed and at the same time the reactivity to the recipient of T cells on the donor side is suppressed, and it is consistent with that donor cells are not rejected in vivo, and GVHD is not caused. Actually, expression of donor MHC class I recognized in the thymus has been recognized not only in mature CD4 single positive and CD8 single positive, but also in CD24(+)CD4(-)CD8(-) T cell that is a pre-T cell (refer to Fig. 5). Thus, it is considered that the donor T cells recognized in a chimeric mouse (recipient mouse after transplantation) are educated in the thymus and then peripherally circulate.

Based on the above, it is suggested that a donor-derived cortical thymic epithelial cell (cTEC) inducing donor T cells in the thymus by positive selection is present, and if so, it is also analogized that negative selection of host T cells by a donor-derived medullary thymicepithelial cell (mTEC) is present at the same time. It is analogized that acquisition of central immune tolerance to the Allo antigen described above is a mechanism of the establishment of a hematopoietic cell chimera using the immune tolerance-inducing agent of the present invention.

### Example 2: Heart transplantation

Ectopic heart transplantation was performed from the same donor (B6 mouse) as the bone marrow donor 14 days after bone marrow transplantation of the TBI+anti-CD40L+KRN7000-containing liposome group and TBI+anti-CD40L group in Example 1. Both donor and recipient mice were anesthetized by intraperitoneally administering Pentobarbital (Dainippon Sumitomo Pharma, Osaka, Japan). The donor mouse was cut opened its abdomen, and 1% heparin physiological saline was injected from the abdominal aorta to perfuse the blood, then the mouse underwent thoracotomy, and the heart was excised. The recipient mouse was cut opened its abdomen, and the ascending aorta and the pulmonary artery of the donor heart were respectively end-to-side anastomosed to the abdominal aorta and the vena cava with 10-0 nylon thread. After the operation, the pulsation of the transplanted heart in the abdomen was manually confirmed, and when the pulsation could not be confirmed, the mouse was cut opened its abdomen, and cardiac arrest was confirmed under direct vision.

The spleen was excised from the euthanatized mouse, then the tissue was ground, and the cells were suspended in CSB. The cell surface antigen was dyed with anti-CD4 and anti-CD25 (BD Bioscience), and the cells were immobilized and made permeabilized using FoxP3 dyeing kit (eBioscience, San Diego, CA, USA), and the nucleus was dyed with anti-FoxP3 and anti-Ki-67 (eBioscience).

Also, for comparison, ectopic heart transplantation was also performed from a donor (C3H mouse) different from the bone marrow donor 14 days after bone marrow transplantation of the TBI+anti-CD40L+KRN7000-containing liposome group in Example 1, and the test was performed in the same conditions as described above.

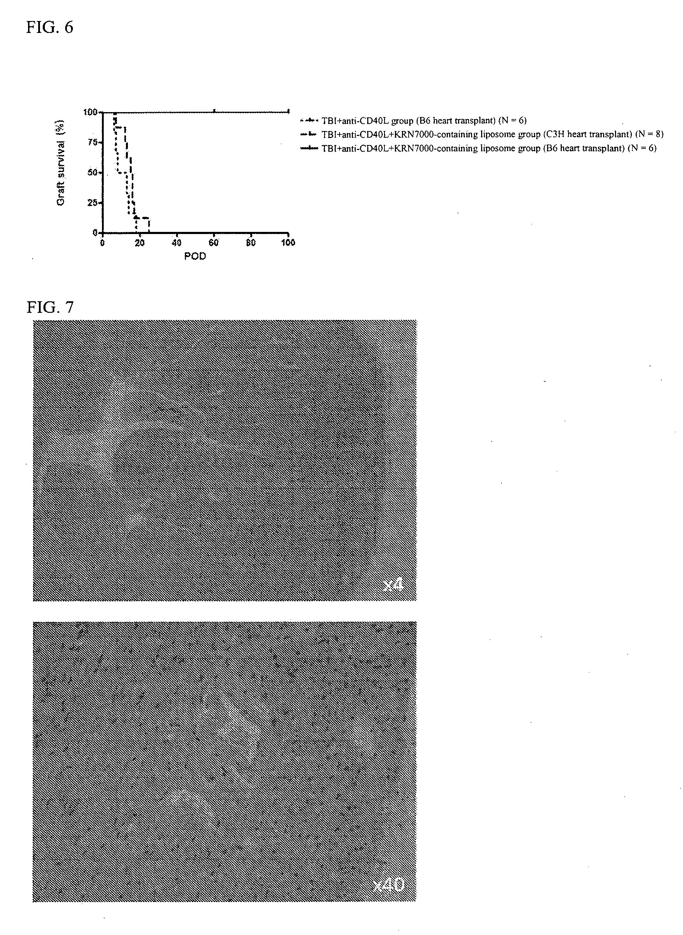

The obtained results are shown in Fig. 6. As is clear from Fig. 6, in the TBI+anti-CD40L group in which a bone marrow chimera was not formed, the graft was rejected in all examples similarly to the bone marrow graft. On the other hand, in the TBI+anti-CD40L+KRN7000-containing liposome group (solid line) in which a bone marrow chimera was successfully formed, the B6 heart graft was engrafted without being rejected. In pathological findings of the graft, cell infiltration was not recognized even after the lapse of 100 days from the transplantation, and vascular lesion suggesting chronic rejection was not also present (refer to Fig. 7). It was also found that this transplanted organ immune tolerance is donor-specific, since the graft was rejected in the TBI+anti-CD40L+KRN7000-containing liposome group (long dotted line) when the C3H heart was transplanted, as in the TBI+anti-CD40L group (fine dotted line).

### Example 3: Evaluation of cytokine production and regulatory T cells after bone marrow transplantation

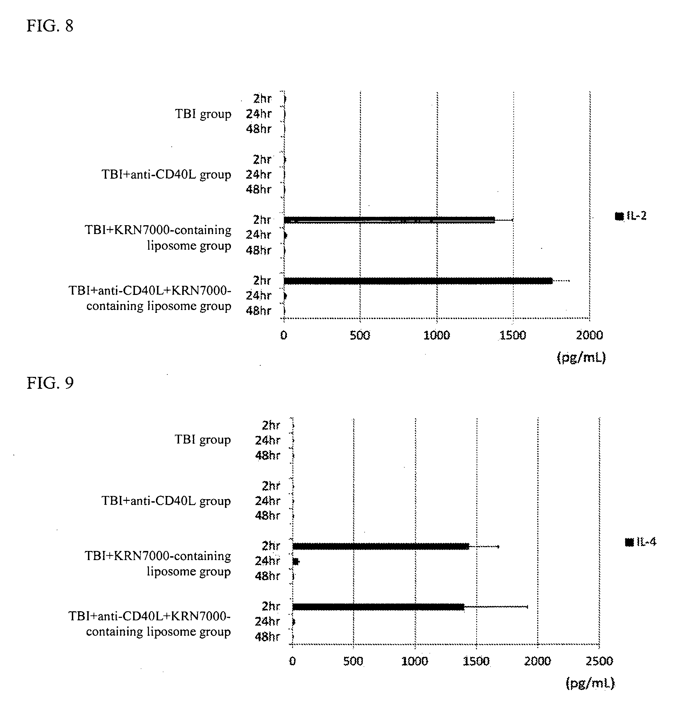

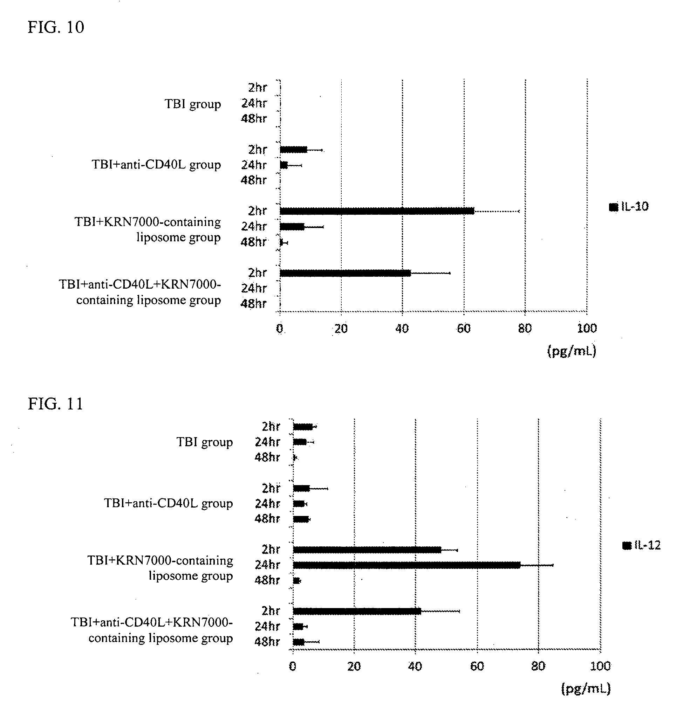

Serum cytokine (IL-2, IL-4, IL-10, IL-12, and IFN-γ) values were measured 2, 24, and 48 hours after bone marrow transplantation for each group in Example 1. The results are shown in Figs. 8 to 12.

IL-10 slightly increased 2 hours after transplantation in the TBI+anti-CD40L group, but was much lower as compared to those in the TBI+anti-CD40L+KRN7000-containing liposome group and the TBI+KRN7000-containing liposome group. However, these cytokines were withdrawn 24 hours after transplantation. IL-12 also rapidly increased after transplantation in the TBI+anti-CD40L+KRN7000-containing liposome group and the TBI+KRN7000-containing liposome group. While IL-12 production was enhanced after 24 hours in the TBI+KRN7000-containing liposome group, IL-12 was withdrawn after 24 hours in the TBI+anti-CD40L+KRN7000-containing liposome group. In the TBI+KRN7000-containing liposome group, following the high IL-12 value after 24 hours, remarkable increase of IFN-γ was recognized. On the other hand, in the TBI+anti-CD40L+KRN7000-containing liposome group, increase of IFN-γ after 24 hours was not recognized. Production of IFN-γ is IL-12-dependent, and IL-12 production is dependent on the costimulatory system of CD40-CD40L, and thus it is considered that blocking of this route by the anti-CD40L antibody suppressed the production of IFN-γ in the TBI+anti-CD40L+KRN7000-containing liposome group.

Based on the above results, it was shown that, due to combined administration of the anti-CD40L antibody and the KRN7000-containing liposome, cytokine production that induces T cells to Th1 was suppressed, and cytokine production that induces to Th2 became dominant.

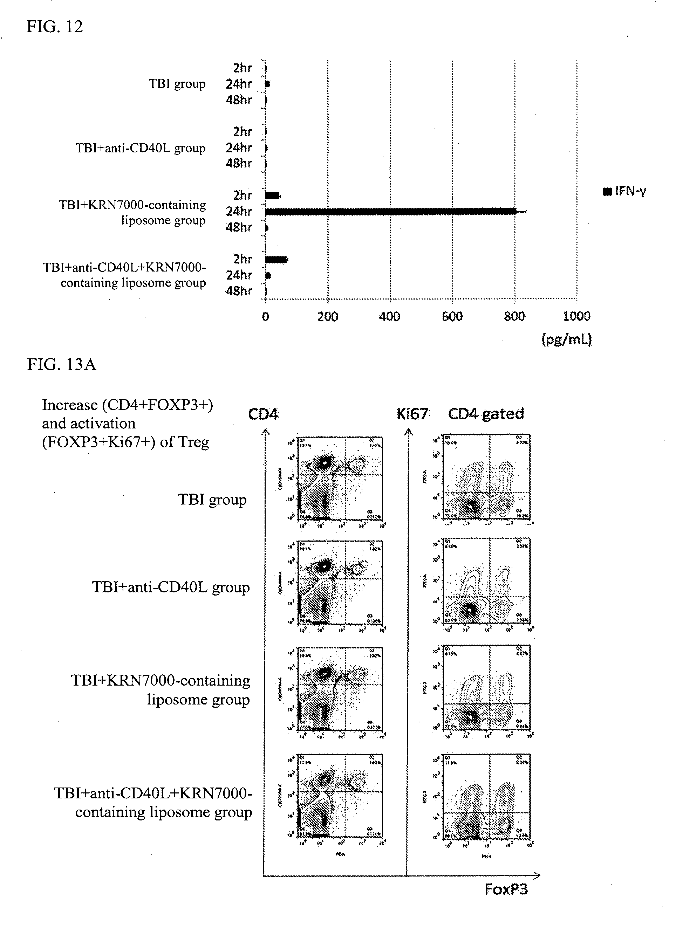

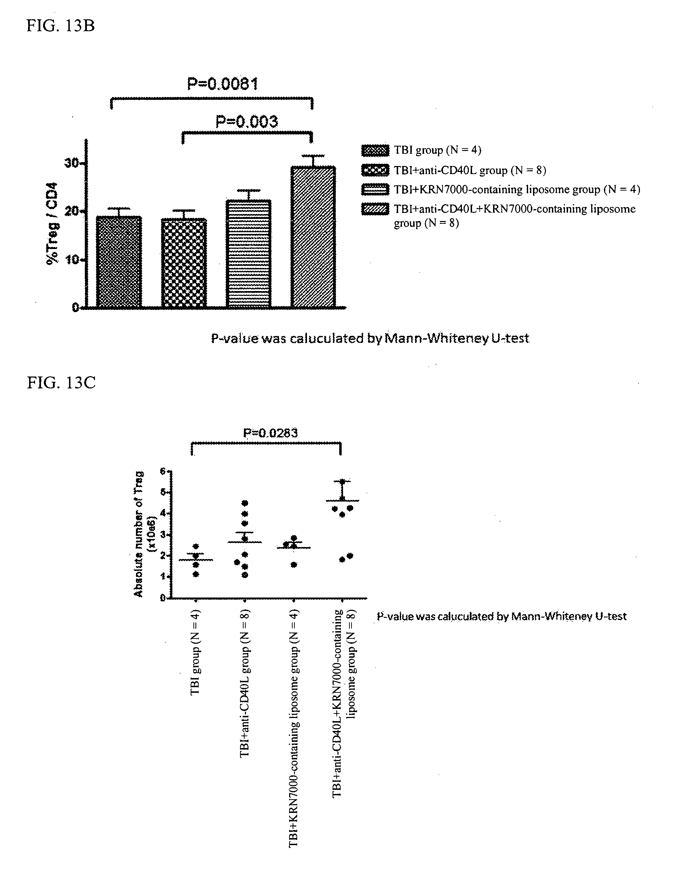

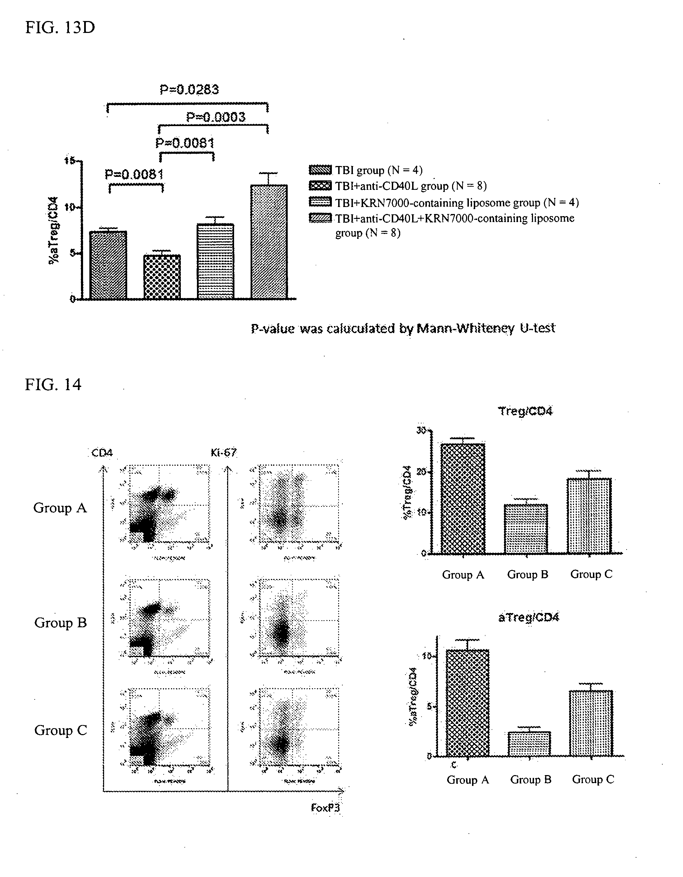

IL-2 and IL-10 that increase in the TBI+anti-CD40L+KRN7000-containing liposome group are known as factors enhancing Treg (regulatory T cells) that plays an important role in immune tolerance. Actually, it was confirmed that Treg in the spleen cells on the 28th day after transplantation was increased in the TBI+anti-CD40L+KRN7000-containing liposome group, as compared to other groups (refer to Figs. 13A, 13B and 13C). Furthermore, it was shown that activated Treg shown by Ki67 positive also significantly increases in the TBI+anti-CD40L+KRN7000-containing liposome group (refer to Figs. 13A and 13D). Based on the above results, it was proved that concurrent use of the anti-CD40L antibody and the KRN7000-containing liposome enables numerical and functional enhancement of Treg.

In addition, in the TBI+anti-CD40L+KRN7000-containing liposome group, the anti-CD25 antibody was administered in the conditions shown in Table 3, and whether or not a bone marrow chimera was established and the number of Treg in the spleen cells were evaluated 14 days after bone marrow transplantation.

**[Table 3]**

| | | Administration timing and administration amount of anti-CD25 antibody |
|---|---|---|
| Group A (n = 4) | Anti-CD25 antibody not administered in TBI+anti-CD40L+KRN7000-containing liposome group | - |
| Group B (n = 4) | Anti-CD25 antibody administered in TBI+anti-CD40L+KRN7000-containing liposome group | Administered at 0.5 mg/head 1 day before bone marrow transplantation and each at 0.25 mg/head 3, 7, 10 and 13 days after bone marrow transplantation |
| Group C (n = 4) | Anti-CD25 antibody administered in TBI+anti-CD40L+KRN7000-containing liposome group | Administered at 0.5 mg/head 14 days after bone marrow transplantation and each at 0.25 mg/head five times at every three days thereafter |

The obtained results are shown in Table 4 and Fig. 14. During 1 to 18 days after bone marrow transplantation, when Treg was deleted by administering the anti-CD25 antibody, a bone marrow chimera was not established (group B), and thus it is assumed that enhancement of Treg is essential for induction of immune tolerance using the anti-CD40L antibody and the KRN7000-containing liposome. Interestingly, even when Treg was deleted by administering the anti-CD25 antibody 28 days after bone marrow transplantation or later, a bone marrow chimera was not released (group C). More specifically, for induction of immune tolerance using the anti-CD40L antibody and the KRN7000-containing liposome, it is considered that maintenance of tolerance by Treg is no longer essential once central tolerance is established.

**[Table 4]**

| | 4 weeks after bone marrow transplantation | |
|---|---|---|
| | Generation ratio of bone marrow chimera | Ratio of donor cells (%) |
| Group A (n = 4) | 4/4 | 71.8 ± 37.1 |
| Group B (n = 4) | 0/4 | - |
| Group C (n = 4) | 4/4 | 78.8 ± 7.4 |

## Claims

1. An immune tolerance-inducing agent used in a therapy in which donor hematopoietic cells are transplanted into a recipient in order to induce immune tolerance in the recipient with respect to donor cells, tissue, or organ, comprising liposome containing α-galactosylceramide and a substance inhibiting costimulatory pathway.

2. The immune tolerance-inducing agent according to claim 1, wherein the costimulatory pathway is CD40/CD40L costimulatory pathway.

3. The immune tolerance-inducing agent according to claim 1 or 2, wherein the substance inhibiting costimulatory pathway is an anti-CD40 antibody or an anti-CD40L antibody.

4. The immune tolerance-inducing agent according to any one of claims 1 to 3, wherein the donor hematopoietic cells to be transplanted are hematopoietic cells derived from bone marrow, cord blood, or peripheral blood.

5. The immune tolerance-inducing agent according to any one of claims 1 to 3, wherein the donor hematopoietic cells to be transplanted are bone marrow.

6. The immune tolerance-inducing agent according to any one of claims 1 to 5, wherein the donor cells, tissue, or organ are a part or the whole of the heart, lung, liver, small intestine, skin, kidney or pancreas derived from the donor.

7. The immune tolerance-inducing agent according to any one of claims 1 to 6, wherein the liposome containing α-galactosylceramide and the substance inhibiting costimulatory pathway are contained in one preparation.

8. The immune tolerance-inducing agent according to any one of claims 1 to 6, which is of a two-agent form comprising a preparation comprising the liposome containing α-galactosylceramide and a preparation comprising the substance inhibiting costimulatory pathway.

9. A method for inducing immune tolerance in a recipient with respect to donor cells, tissue, or organ, comprising administering liposome containing α-galactosylceramide and a substance inhibiting costimulatory pathway to the recipient into which donor hematopoietic cells were transplanted or the recipient into which donor hematopoietic cells are to be transplanted.

10. Use of liposome containing α-galactosylceramide and a substance inhibiting costimulatory pathway for producing an immune tolerance-inducing agent used in a therapy in which donor hematopoietic cells are transplanted into a recipient in order to induce immune tolerance in the recipient with respect to donor cells, tissue, or organ.

11. A hematopoietic cell chimeria-inducing agent, comprising liposome containing α-galactosylceramide and a substance inhibiting costimulatory pathway.

12. The hematopoietic cell chimeria-inducing agent according to claim 11, which is administered to a recipient into which donor hematopoietic cells were transplanted or a recipient into which donor hematopoietic cells are to be transplanted.

13. The hematopoietic cell chimeria-inducing agent according to claim 12, wherein the donor hematopoietic cells are hematopoietic cells derived from bone marrow, cord blood, or peripheral blood.

14. The hematopoietic cell chimeria-inducing agent according to any one of claims 11 to 13, wherein the costimulatory pathway is CD40/CD40L costimulatory pathway.

15. The hematopoietic cell chimeria-inducing agent according to any one of claims 11 to 14, wherein the substance inhibiting costimulatory pathway is an anti-CD40 antibody or an anti-CD40L antibody.

16. A method for inducing formation of a hematopoietic cell chimera in the body of a recipient, comprising administering liposome containing α-galactosylceramide and a substance inhibiting costimulatory pathway to the recipient into which donor hematopoietic cells were transplanted or the recipient into which donor hematopoietic cells are to be transplanted.

17. Use of liposome containing α-galactosylceramide and a substance inhibiting costimulatory pathway for producing a hematopoietic cell chimeria-inducing agent.
